(19) Europäisches Patentamt
European Patent Office
Office européen des brevets

(11) **EP 4 197 539 A1**

(12) **EUROPEAN PATENT APPLICATION**
published in accordance with Art. 153(4) EPC

(43) Date of publication:
**21.06.2023 Bulletin 2023/25**

(21) Application number: **21868634.3**

(22) Date of filing: **15.09.2021**

(51) International Patent Classification (IPC):
***A61K 31/519*** *(2006.01)* ***A61P 35/00*** *(2006.01)*

(52) Cooperative Patent Classification (CPC):
**A61K 31/519; A61P 35/00**

(86) International application number:
**PCT/CN2021/118417**

(87) International publication number:
**WO 2022/057812 (24.03.2022 Gazette 2022/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.09.2020 CN 202010967168**

(71) Applicant: **Chia Tai Tianqing Pharmaceutical Group Co., Ltd.**
**Lianyungang City, Jiangsu 222062 (CN)**

(72) Inventors:
- **FENG, Fan**
  **Nanjing City, Jiangsu 211100 (CN)**
- **WANG, Xunqiang**
  **Nanjing City, Jiangsu 211100 (CN)**
- **ZHAO, Ying**
  **Nanjing City, Jiangsu 211100 (CN)**
- **HAN, Xi**
  **Nanjing City, Jiangsu 211100 (CN)**
- **CHEN, Li**
  **Nanjing City, Jiangsu 211100 (CN)**
- **MA, Ruiting**
  **Nanjing City, Jiangsu 211100 (CN)**
- **WU, Naiying**
  **Nanjing City, Jiangsu 211100 (CN)**

(74) Representative: **Müller Schupfner & Partner Patent- und Rechtsanwaltspartnerschaft mbB**
**Bavariaring 11**
**80336 München (DE)**

(54) **USE OF PYRIDO[1,2-A]PYRIMIDINONE COMPOUND IN TREATING PERIPHERAL T CELL LYMPHOMA**

(57) A pyrido[1,2-a]pyrimidinone compound or a pharmaceutical composition thereof for treating peripheral T cell lymphoma, and a method for or use of a pyrido[1,2-a]pyrimidinone compound for treating peripheral T cell lymphoma.

EP 4 197 539 A1

## Description

### CROSS-REFERENCE TO RELATED APPLICATIONS

The present application claims priority and benefit to the Chinese Patent Application No. 202010967168.5 filed with National Intellectual Property Administration, PRC on Sep. 15, 2020, the disclosure of which is incorporated herein by reference in its entirety.

### TECHNICAL FIELD

The present application belongs to the field of medicinal chemistry, and relates to use of a pyrido[1,2-a]pyrimidinone compound in treating peripheral T-cell lymphoma.

### BACKGROUND

PI3K pathway is the most frequently mutated part in cancer cells of the human body, which can lead to proliferation, activation and signal amplification of cells.

PI3K kinase (phosphatidylinositol-3-kinase, PI3Ks) belongs to the lipid kinase family and can phosphorylate 3'-OH end of the inositol ring of phosphatidylinositol. The PI3K kinase is a lipid kinase consisting of a regulatory subunit p85 or p101 and a catalytic subunit p110, and activates downstream Akt and the like by catalyzing phosphorylation of phosphatidylinositol 4,5-bisphosphate (PIP2) to phosphatidylinositol 3,4,5-trisphosphate (PIP3), thereby playing a key role in proliferation, survival, metabolism and the like of cells. Therefore, inhibiting the phosphatidylinositol-3-kinase may affect the PI3K pathway, thereby inhibiting proliferation and activation of cancer cells.

The tumor suppressor gene PTEN (phosphatase and tension homolog deleted on chromosome ten) enables PIP3 to be dephosphorylated to generate PIP2, thereby achieving negative regulation of PI3K/Akt signaling pathway, inhibiting proliferation of cells and promoting apoptosis of the cells. The frequent occurrences of PI3K gene mutation and amplification in cancers, absence of PTEN in cancers and the like all suggest that PI3K is closely related to tumorigenesis.

A series of compounds as PI3K inhibitors are disclosed in WO2015192760, and a compound of formula **I** with the following structure is also specifically disclosed:

I .

### BRIEF SUMMARY

The present application provides a compound of formula **I** or a pharmaceutically acceptable salt thereof for use in treating peripheral T-cell lymphoma in a patient:

I

In another aspect, the present application provides use of the compound of formula **I** or the pharmaceutically acceptable salt thereof for preparing a medicament for treating peripheral T-cell lymphoma in a patient.

In another aspect, the present application provides use of the compound of formula **I** or the pharmaceutically acceptable salt thereof in treating peripheral T-cell lymphoma in a patient.

In another aspect, the present application provides a method for treating peripheral T-cell lymphoma in a patient, which comprises administering to the patient an effective amount of the compound of formula **I** or the pharmaceutically acceptable salt thereof.

In some embodiments of the present application, the compound of formula **I** or the pharmaceutically acceptable salt thereof disclosed herein is used as a single active agent.

In some embodiments of the present application, the compound of formula **I** or the pharmaceutically acceptable salt thereof may be a pharmaceutical composition comprising a therapeutically effective amount of the compound of formula **I** or the pharmaceutically acceptable salt thereof. In some embodiments, the pharmaceutical composition is a pharmaceutical composition in single doses.

In another aspect, the present application provides a pharmaceutical composition for use in treating peripheral T-cell lymphoma, which comprises the compound of formula **I** or the pharmaceutically acceptable salt thereof.

In another aspect, the present application provides a method for treating peripheral T-cell lymphoma in a patient, which comprises administering to the patient a therapeutically effective amount of the compound of formula **I** or the pharmaceutically acceptable salt thereof, or the pharmaceutical composition thereof.

In another aspect, the present application provides a kit for use in treating peripheral T-cell lymphoma, which comprises the compound of formula **I** or the pharmaceutically acceptable salt thereof, or the pharmaceutical composition thereof described herein, preferably in single dose form, and instructions.

## DETAILED DESCRIPTION OF INVENTION

### Peripheral T Cell Lymphoma

In some embodiments of the present application, the peripheral T-cell lymphoma is selected from relapsed or refractory peripheral T-cell lymphoma.

In some embodiments of the present application, the patient with the peripheral T-cell lymphoma has been treated with one or more prior treatment regimens. In some embodiments of the present application, the patient with the peripheral T-cell lymphoma has been treated with one, two, three, four or five prior treatment regimens.

In some embodiments of the present application, the patient with the peripheral T-cell lymphoma is one who has been treated with a first-line, second-line or ≥ third-line prior treatment regimen.

In some embodiments of the present application, the disease reoccurs after the patient with the peripheral T-cell lymphoma has been treated with prior treatment regimens and achieved objective response, or the patient with the peripheral T-cell lymphoma has been treated with prior treatment regimens but achieved no objective response. In some embodiments of the present application, the no objective response refers to stable disease or disease progression during treatment.

In some embodiments of the present application, the patient with the peripheral T-cell lymphoma is one who has been treated with a ≥ first-line systemic treatment regimen but had no objective response (stable disease or disease progression during the treatment) to the latest treatment regimen used or had disease progression after the treatment.

In some embodiments of the present application, the patient with the peripheral T-cell lymphoma is a patient with relapsed or refractory T-cell lymphoma who has been treated with prior treatment regimens.

In some embodiments of the present application, the patient with the peripheral T-cell lymphoma is a patient who has been treated with one or more prior treatment regimens comprising pegaspargase or L-asparaginase.

In some embodiments of the present application, the prior treatment regimens include drug therapies, radiotherapy or hematopoietic stem cell transplantation.

In some embodiments of the present application, the drug therapies of the prior treatment regimens include interferon therapies, chemotherapy or targeted drug therapies.

In some embodiments of the present application, drugs used for the chemotherapy of the prior therapeutic regimens include pegaspargase, asparaginase (e.g., L-asparaginase), cyclophosphamide, ifosfamide, vincristine, vindesine, prednisone, prednisolone, doxorubicin, adriamycin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil or azacitidine.

In some embodiments of the present application, the targeted drug therapies of the prior treatment regimens include anti-tumor folic acid analog therapies, histone deacetylase inhibitor therapies, proteasome inhibitor therapies, immunomodulatory inhibitor therapies or immune checkpoint inhibitor therapies.

In some embodiments of the present application, the anti-tumor folic acid analogs include pralatrexate.

In some embodiments of the present application, the histone deacetylase inhibitors include romidepsin, belinostat or chidamide.

In some embodiments of the present application, the proteasome inhibitors include bortezomib.

In some embodiments of the present application, the immunomodulatory inhibitors include lenalidomide or thalidomide.

In some embodiments of the present application, the immune checkpoint inhibitors include programmed death receptor-1 (PD-1) inhibitors and programmed death ligand-1 (PD-L1) inhibitors. In some embodiments of the present application, the immune checkpoint inhibitors include genolimzumab, cemiplimab, pembrolizumab, nivolumab, sintilimab, tislelizumab, avelumab or atezolizumab.

In some embodiments of the present application, the targeted drugs of the prior treatment regimens include pralatrexate, romidepsin, belinostat, chidamide, bortezomib, lenalidomide, thalidomide, genolimzumab, cemiplimab, pembrolizumab, nivolumab, sintilimab, tislelizumab, avelumab or atezolizumab.

In some embodiments of the present application, the drugs used in the drug therapies of the prior treatment regimens include one of pegaspargase, asparaginase (e.g., L-asparaginase), cyclophosphamide, ifosfamide, vincristine, vindesine, prednisone, prednisolone, doxorubicin, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil, azacitidine, pralatrexate, romidepsin, belinostat, chidamide, bortezomib, lenalidomide, thalidomide, calcium folinate, rituximab, genolimzumab, cemiplimab, pembrolizumab, nivolumab, sintilimab, tislelizumab, avelumab, atezolizumab and G-CSF, or combinations of more than one of the drugs described above; preferably, the drugs used in the drug therapies of the prior treatment regimens include one of pegaspargase, L-asparaginase, cyclophosphamide, ifosfamide, vincristine, vindesine, prednisone, prednisolone, doxorubicin, adriamycin, dexamethasone, methotrexate, cytarabine, carboplatin, cisplatin, bendamustine, fludarabine, mitoxantrone, etoposide, procarbazine, gemcitabine, methylprednisolone sodium succinate, mesna, oxaliplatin, 5-fluorouracil, pralatrexate, romidepsin, belinostat, chidamide, bortezomib, lenalidomide, genolimzumab, cemiplimab, pembrolizumab, nivolumab, sintilimab, tislelizumab, avelumab, atezolizumab and G-CSF, or combinations of more than one of the drugs described above.

In some embodiments of the present application, the chemotherapy regimens of the prior treatment regimens include AOEP regimen, AOEP+G-CSF regimen, AspaMetDex regimen, B regimen, BAC regimen, CHOP regimen, miniCHOP regimen, CHOEP regimen, CHOEP-chidamide combination regimen, CIFOX regimen, COP regimen, COEP-L regimen, DHAP regimen, DDGP regimen, EPOCH regimen, DA-EPOCH regimen, ESHAP regimen, GDP regimen, GDPE regimen, GEMOX regimen, FC regimen, FM regimen, HyperCVAD regimen, ICE regimen, LOP regimen, MA regimen, P-GEMOX regimen, SMILE regimen, V-CAP regimen, or combinations of the regimens described above and rituximab (referred to hereinafter as "R"); preferably, the chemotherapy regimens of the prior treatment regimens include AOEP regimen, AOEP+G-CSF regimen, AspaMetDex regimen, R-HyperCVAD regimen, BR regimen, CHOP regimen, R-CHOP regimen, R-miniCHOP regimen, CHOEP regimen, COP regimen, COEP-L regimen, DHAP regimen, R-DHAP regimen, DA-EPOCH regimen, DA-EPOCH-R regimen, DDGP regimen, ESHAP regimen, FCR regimen, FMR regimen, GDP regimen, $R^2$ regimen, R-GDP regimen, R-GDPE regimen, GEMOX regimen, HyperCVAD regimen, ICE regimen, R-ICE regimen, LOP regimen, VR-CAP regimen, R-GEMOX regimen, P-GEMOX regimen or R-high-dose cytarabine regimen.

In some embodiments of the present application, the radiotherapy of the prior treatment regimens is selected from the group consisting of total lymphoid irradiation (TLI) and sub-total lymphoid irradiation (STLI). In some embodiments of the present application, the radiotherapy includes involved field radiation therapy (IFRT), involved nodal radiation therapy (INRT) or involved site radiation therapy (ISRT).

In some embodiments of the present application, the hematopoietic stem cell transplantation of the prior treatment regimens includes autologous hematopoietic stem cell transplantation or allogeneic hematopoietic stem cell transplantation.

In some embodiments of the present application, the peripheral T-cell lymphoma is selected from the group consisting of peripheral T-cell lymphoma, not otherwise specified (PTCL-NOS); angioimmunoblastic T-cell lymphoma (AITL); anaplastic large cell lymphoma (ALCL); and extranodal NK/T cell lymphoma (NKTCL), nasal type.

In some embodiments of the present application, the peripheral T-cell lymphoma is selected from peripheral T-cell lymphoma, not otherwise specified (PTCL-NOS).

In some embodiments of the present application, the peripheral T-cell lymphoma, not otherwise specified is selected from the group consisting of a GATA3-overexpressing type, a TBX21-overexpressing type, and a cytotoxin-overexpressing genotype.

In some embodiments of the present application, the peripheral T-cell lymphoma is selected from relapsed or refractory peripheral T-cell lymphoma; optionally, the patient with the peripheral T-cell lymphoma has been treated with one or more prior treatment regimens; optionally, the disease reoccurs after the patient with the peripheral T-cell lymphoma has been treated with prior treatment regimens and achieved objective response, or the patient with the peripheral T-cell lymphoma has been treated with prior treatment regimens but achieved no objective response; optionally, the prior treatment regimens include drug therapies, radiotherapy or hematopoietic stem cell transplantation.

## Administration regimen

In some embodiments of the present application, an administration cycle for treating peripheral T-cell lymphoma in a patient is 2-6 weeks. In some embodiments of the present application, the administration cycle for treating peripheral T-cell lymphoma in a patient is 2 weeks, 3 weeks, 4 weeks, 5 weeks, 6 weeks, or a range formed by any of the aforementioned values. In some embodiments of the present application, the administration cycle for treating peripheral T-cell lymphoma in a patient is 3 weeks.

In some embodiments of the present application, a daily dose for treating peripheral T-cell lymphoma in a patient is selected from 1-100 mg. In some embodiments of the present application, the daily dose for treating peripheral T-cell lymphoma in a patient is selected from the group consisting of 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg, and ranges formed by any of the aforementioned values. In some embodiments of the present application, the daily dose for treating peripheral T-cell lymphoma in a patient is selected from the group consisting of 1-50 mg, 5-50 mg, 10-50 mg, 10-40 mg and 20-40 mg.

In some embodiments of the present application, the number of daily administrations for treating peripheral T-cell lymphoma in a patient is 1, 2 or 3.

In some embodiments of the present application, the administration for treating peripheral T-cell lymphoma in a patient is performed once every two days.

In some embodiments of the present application, the administration regimen for treating peripheral T-cell lymphoma in a patient includes: an administration cycle of 2-6 weeks, a daily dose of 1-40 mg, and 1-3 administrations daily.

The compound of formula **I** or the pharmaceutically acceptable salt thereof, or the pharmaceutical composition thereof, disclosed herein can be administered via multiple routes including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular and intrathecal administrations. In specific embodiments, the route is oral administration.

The route of administration can be determined according to factors such as the activity and toxicity of the drug, and tolerance of the patient. In some embodiments, the compound of formula **I** or the pharmaceutically acceptable salt thereof, or the pharmaceutical composition thereof, disclosed herein is administered at intervals.

## Pharmaceutical composition

The pharmaceutical composition of the present application can be prepared by combining the compound of formula I or the pharmaceutically acceptable salt thereof disclosed herein with a suitable pharmaceutically acceptable excipient/carrier, and can be formulated into, for example, a solid, semisolid, liquid or gaseous preparation.

In some embodiments of the present application, the pharmaceutical composition is a preparation suitable for oral administration, including tablets, capsules, powders, granules, dripping pills, pastes, pulvis, and the like, preferably tablets and capsules. The oral preparation may be prepared by a conventional method using a pharmaceutically acceptable carrier well known in the art. The pharmaceutically acceptable carrier includes diluents, binders, wetting agents,

disintegrants, lubricants, and the like.

In some embodiments of the present application, the pharmaceutical composition is a pharmaceutical composition in single doses. In some embodiments, the pharmaceutical composition comprises 1 mg to 50 mg of the compound of formula **I** or the pharmaceutically acceptable salt thereof disclosed herein. In some embodiments, the pharmaceutical composition comprises 1 mg, 2 mg, 5 mg, 8 mg, 10 mg, 12 mg, 15 mg, 18 mg, 20 mg, 22 mg, 25 mg, 28 mg, 30 mg, 32 mg, 35 mg, 38 mg, 40 mg, 42 mg, 45 mg, 48 mg or 50 mg, or a range of any two of the foregoing values as endpoints or any value therein, of the compound of formula **I** or the pharmaceutically acceptable salt thereof disclosed herein, for example, 2 mg to 50 mg, 10 mg to 40 mg, 5 mg to 30 mg, or 5 mg to 20 mg.

## Technical Effects

The compound of formula I or the pharmaceutically acceptable salt thereof, or the pharmaceutical composition thereof, disclosed herein has favorable therapeutic efficacy in reducing the growth of peripheral T-cell lymphoma or even eliminating tumors, and provides good disease control rate (DCR) to the treated patients to allow them to have longer survival (e.g., median survival, progression-free survival or overall survival), and longer duration of response (DOR) for the disease. Meanwhile, the compound of formula I or the pharmaceutically acceptable salt thereof, or the pharmaceutical composition thereof, disclosed herein has good safety while reducing the growth of peripheral T-cell lymphoma.

## Definitions and Description

Unless otherwise stated, the following terms used herein shall have the following meanings. A certain term, unless otherwise specifically defined, should not be considered uncertain or unclear, but construed according to its common meaning in the field. When referring to a trade name, it is intended to refer to its corresponding commercial product or its active ingredient.

As used herein, unless otherwise stated, the terms "comprise", "comprises" and "comprising" or equivalents thereof are open-ended statements and mean that elements, components and steps that are not specified may be included in addition to those listed.

All patents, patent applications and other identified publications are explicitly incorporated herein by reference for the purpose of description and disclosure. Any reference to these publications herein shall not be construed as an admission that the publications form part of the commonly recognized knowledge in the art.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed from basic radicals and free acids and salts formed from acidic radicals and free bases.

As used herein, the amount of the compound of formula **I** or the pharmaceutically acceptable salt thereof, e.g., the amount administered, the dose or the amount in the pharmaceutical composition, is calculated based on its free base form.

As used herein, if the compound in the pharmaceutical combination has, for example, at least one basic site, an acid addition salt may be formed. If needed, it may further form an acid addition salt with additional existing basic sites. A compound with at least one acidic group (for example, -COOH) can further form a salt with a base. A compound, for example, comprising both carboxyl and amino, can further form an inner salt.

The term "patient" is a mammal. In some embodiments, the patient is a human.

The term "pharmaceutical composition" refers to a mixture consisting of one or more of the compounds of formula I or the pharmaceutically acceptable salts thereof, or the pharmaceutical combinations thereof, disclosed herein and a pharmaceutically acceptable excipient/carrier. The pharmaceutical composition is intended to facilitate the administration of the compound or the therapeutic combination thereof disclosed herein to a patient. The term "single dose" refers to the smallest unit of packaging containing a certain quantity of pharmaceutical product; for example, each tablet of drug is a single dose; in a box of seven capsules, each capsule is a single dose; or a vial of injection is a single dose.

The term "treat", "treating" or "treatment" usually refers to acquiring needed pharmacological effect and/or physiological effect. In terms of partially or fully stabilizing or curing the disease and/or a side effect of the disease, the effect can be therapeutic. As used herein, "treat", "treating" or "treatment" encompasses any treatment of a disease in a patient, including (a) inhibiting a symptom of the disease, i.e., blocking the progression of the disease; or (b) alleviating a symptom of the disease, i.e., causing remission of the disease or the symptom.

The term "effective amount" refers to an amount of the compound disclosed herein for (i) treating a specific disease, condition or disorder; (ii) alleviating, relieving or eliminating one or more symptoms of a specific disease, condition or disorder, or (iii) preventing or delaying onset of one or more symptoms of a specific disease, condition or

disorder described herein. The amount of the compound disclosed herein composing the "therapeutically effective amount" varies dependently on the compound, the condition and its severity, the administration regimen, and the age of the mammal to be treated, but can be determined routinely by those skilled in the art in accordance with their knowledge and the present disclosure.

In the context of cancer, the term "refractory" means that a particular cancer is resistant or non-responsive to therapy with a particular therapeutic agent. Cancers that are refractory to therapy with a particular therapeutic agent can begin when treatment with that particular therapeutic agent begins (i.e., does not respond as soon as exposure to the therapeutic agent begins), or develop resistance to the therapeutic agent during the first treatment period with the therapeutic agent or during subsequent treatments with the therapeutic agent.

In the context of cancer, the term "relapsed" means that a disease reoccurs after objective response is achieved through treatment with a certain treatment regimen. "Objective response" includes complete response and partial response.

In the context of cancer, the term "first-line therapy" refers to the first treatment of a disease. It is usually part of a standard set of treatments, such as post-operative chemotherapy and radiotherapy. The first-line therapy, when used alone, is recognized as the best therapy. If it does not cure the disease or causes serious side effects, other treatment methods may be added or used.

As used herein, with respect to drugs used in the prior therapy, reference may be made to the following, or treatment guidelines or textbooks relating to medicine and pharmacy:

AOEP regimen: cytarabine, vindesine, etoposide and dexamethasone;
AspaMetDex regimen: asparaginase, methotrexate and dexamethasone;
B regimen: bendamustine;
BAC regimen: bendamustine and cytarabine;
BR regimen: bendamustine and rituximab;
CHOP regimen: cyclophosphamide, adriamycin/epirubicin, vincristine and prednisone; the CHOP regimen includes, but is not limited to, a CHOP-21 day regimen or a CHOP-14 day regimen;
miniCHOP regimen: dose-reduction CHOP (dose reduced to 1/2 to 1/3 of the standard dose);
CHOEP regimen: cyclophosphamide, adriamycin/epirubicin, vincristine, etoposide and prednisone (CHOP regimen in combination with etoposide);
CIFOX regimen: 5-fluorouracil and oxaliplatin;
COEP-L regimen: cyclophosphamide, vincristine (or vindesine), etoposide, prednisone (or dexamethasone) and pegaspargase;
COP regimen: cyclophosphamide, vincristine and prednisone;
EPOCH regimen: etoposide, prednisone, vincristine, cyclophosphamide and adriamycin;
DA-EPOCH regimen (dose-adjustment EPOCH): etoposide, prednisone, vincristine, cyclophosphamide and adriamycin;
DA-EPOCH-R regimen (dose-adjustment EPOCH-R): etoposide, prednisone, vincristine, cyclophosphamide, adriamycin and rituximab;
DDGP regimen: dexamethasone, cisplatin, gemcitabine and pegaspargase;
DHAP regimen: dexamethasone, high-dose cytarabine and cisplatin;
ESHAP regimen: etoposide, methylprednisolone, high-dose cytarabine and cisplatin;
FC regimen: fludarabine and cyclophosphamide;
FCR regimen: fludarabine, cyclophosphamide and rituximab;
FM regimen: fludarabine and mitoxantrone;
FMR regimen: fludarabine, mitoxantrone and rituximab;
G-CSF: granulocyte-colony stimulating factor;
GDP regimen: gemcitabine, dexamethasone and cisplatin;
GDPE regimen: gemcitabine, dexamethasone, cisplatin and etoposide;
GEMOX regimen: gemcitabine and oxaliplatin;
HyperCVAD regimen: regimen A: cyclophosphamide, mesna, vincristine, adriamycin and dexamethasone; regimen B: methotrexate and cytarabine;
ICE regimen: ifosfamide, carboplatin and etoposide;
LOP regimen: pegaspargase, vincristine and prednisone;
MA regimen: methotrexate and cytarabine;
P-GEMOX regimen: pegaspargase, gemcitabine and oxaliplatin;
R regimen: rituximab;
$R^2$ regimen: rituximab + lenalidomide;
R-: refers to the combination of rituximab with a treatment regimen. It includes, but is not limited to, the following:

R-CHOP regimen: rituximab, cyclophosphamide, adriamycin/epirubicin, vincristine and prednisone;
R-miniCHOP regimen: rituximab and dose-reduction CHOP (dose reduced to 1/2 to 1/3 of the standard dose);
R-DHAP regimen: rituximab, dexamethasone, cytarabine and cisplatin;
R-GDP regimen: rituximab, gemcitabine, dexamethasone and cisplatin;
R-GDPE regimen: rituximab, gemcitabine, dexamethasone, cisplatin and etoposide;
R-GEMOX regimen: rituximab, gemcitabine and oxaliplatin;
R-HyperCVAD regimen: regimen A: rituximab, cyclophosphamide, mesna, vincristine, adriamycin and dexamethasone; regimen B: rituximab, methotrexate and cytarabine;
R-ICE regimen: rituximab, ifosfamide, carboplatin and etoposide.
SMILE regimen: dexamethasone, methotrexate, calcium folinate, mesna, ifosfamide, L-asparaginase and etoposide (according to the clinical practical medication, SMILE regimen may also be: dexamethasone, methotrexate, ifosfamide, pegaspargase and etoposide).
V-CAP regimen: bortezomib, cyclophosphamide, adriamycin and prednisone.
VR-CAP regimen: bortezomib, rituximab, cyclophosphamide, adriamycin and prednisone.
R-high-dose cytarabineregimen: rituximab and high-dose cytarabine.

As used herein, the prednisolone may also be prednisone, and the two are used interchangeably.
As used herein, adriamycin may also be doxorubicin, and the two are used interchangeably.
As used herein, the chemotherapy regimens belong to the prior art in this field. Those skilled in the art would readily refer to treatment guidelines or related medical and pharmaceutical textbooks in the prior art (e.g., Chinese Society of Clinical Oncology (CSCO) diagnosis and treatment guidelines for malignant lymphoma 2019) for details of a chemotherapy regimen (including but not limited to the drug used, the administration dose, or administration cycle). The above examples of drugs used for the chemotherapy regimens in the present application are exemplary, and the details of the chemotherapy regimens (including but not limited to the drug used, the administration dose, or administration cycle) will be subject to treatment guidelines or related medical and pharmaceutical textbooks.

## DETAILED DESCRIPTION

The present invention will be illustrated in more detail through specific examples. The following examples are provided for illustrative purposes only, and are not intended to limit the present invention in any way.

### Example 1 Tablets of the compound of formula I

Table 1 Formulation of tablets of the compound of formula **I**

| Composition | Amount | | | | | |
|---|---|---|---|---|---|---|
| | Specification 5 mg | | Specification 20 mg | | Specification 1 mg | |
| | mg | % | mg | % | mg | % |
| Compound of formula **I** | 5.0 | 5.0 | 20.0 | 5.0 | 1.0 | 1.0 |
| Microcrystalline cellulose | 25.0 | 25.0 | 100.0 | 25.0 | 25.0 | 25.0 |
| Mannitol | 63.0 | 63.0 | 252.0 | 63.0 | 67.0 | 67.0 |
| Croscarmellose sodium | 5 | 5 | 20 | 5 | 5 | 5 |
| Hydroxypropyl methylcellulose | 1.0 | 1.0 | 4.0 | 1.0 | 1.0 | 1.0 |
| Magnesium stearate | 1.0 | 1.0 | 4.0 | 1.0 | 1.0 | 1.0 |
| Weight of core tablet | 100 | 100 | 400 | 100 | 100 | 100 |

Preparation method:

1) The compound of formula **I**, microcrystalline cellulose, mannitol and croscarmellose sodium were each fed into a grinding and sizing machine successively and then sieved, and the materials were then collected and premixed

to obtain a premixed material.

2) Hydroxypropyl methylcellulose was formulated into an aqueous solution to be used as a binder.

3) The premixed material in the step 1) was transferred into a wet granulation pot, the binder obtained in the step 2) was added, and the granulation was started.

4) The soft and wet materials obtained were subjected to sizing and drying, and then magnesium stearate was added to be mixed together.

5) Tableting was performed.

Optionally, the resulting tablets were coated.

The compound of formula I is prepared according to the method disclosed in Patent No. WO2015192760.

**Example 2 Clinical Trials on Peripheral T-Cell Lymphoma**

2.1 Administration regimen

Method of administration: orally administered once daily, 15 mg or 20 mg each time, with 21 consecutive days of administration as one cycle.

Drug: tablet of the compound of formula **I**, 5 mg or 20 mg.

2.2 Enrollment criteria

1) Histopathologically confirmed relapsed/refractory peripheral T-cell lymphoma (PTCL), including four subtypes: peripheral T-cell lymphoma, not otherwise specified (PTCL-NOS); angioimmunoblastic T-cell lymphoma (AITL); anaplastic large cell lymphoma (ALCL); and extranodal NK/T cell lymphoma (NKTCL), nasal type;

2) Has previously received at least first-line systemic treatment regimen, had disease progression during or after the most recent treatment, or had no objective response after adequate treatment;

3) At least one measurable target lesion present (evaluated according to the Lugano evaluation criteria, 2014 edition);

4) Age ≥ 18 years; ECOG (PS) score: 0-2; expected survival time ≥ 3 months;

5) Main organ functions in the screening phase meet the following criteria:

Criteria for blood routine examination (no growth factor used or no blood transfusion conducted within 14 days):

$$\text{Absolute neutrophil count (ANC)} \geq 1.0\times10^9/\text{L};$$

$$\text{Lymphocyte count (LYM)} \geq 0.5\times10^9/\text{L};$$

$$\text{CD4+ T lymphocyte count} \geq 0.2\times10^9/\text{L};$$

$$\text{Platelet (PLT)} \geq 75\times10^9/\text{L (for patients with lymphoma bone marrow infiltration, } \geq 50\times10^9/\text{L acceptable)};$$

$$\text{Hemoglobin (Hb)} \geq 80\ \text{g/L};$$

Criteria for blood biochemical examination:

Alanine transaminase (ALT) and aspartate transferase (AST) ≤ 2.5 × ULN (for patients with liver involvement of lymphoma or biliary obstruction, ≤ 5 × ULN);

Serum total bilirubin (TBIL) ≤ 1.5 × ULN;

Blood coagulation: activated partial thromboplastin time (APTT), international normalized ratio (INR), prothrombin time (PT) ≤ 1.5 × ULN;

Serum creatinine (Cr) ≤ 1.5 × ULN or creatinine clearance ≥ 50 mL/min;

6) Female subjects should agree to take contraceptive measures (such as intrauterine devices [IUD], contraceptives or condoms) during the study and for 6 months after the study; serum or urine pregnancy test results should be negative within 7 days before enrollment, and the patients must not be breastfeeding; male subjects should agree to take contraceptive measures during the study and for 6 months after the study;

7) Voluntary participation, written informed consent and good compliance.

2.3 Evaluation method and index

The efficacy was evaluated according to the revised evaluation criteria of the Lugano conference, 2014 edition. Primary evaluation indexes of efficacy: objective response rate (ORR), i.e., (CR + PR cases)/total cases, including cases of complete response (CR) and partial response (PR).

Secondary evaluation indexes of efficacy: progression-free survival (PFS), overall survival (OS), disease control rate (DCR) and duration of response (DOR).

2.4 Results of trial

In one set of experiments, the efficacy has so far been evaluated in 6 enrolled patients with peripheral T-cell lymphoma, and the objective response rate (ORR) is 50% (3/6). The results indicate that the compound of formula I has a good therapeutic effect on peripheral T-cell lymphoma. Meanwhile, the compound of formula I has good safety in treatment.

In another set of experiments, the efficacy in patients has so far shown that the compound of formula I has a good therapeutic effect on peripheral T-cell lymphoma. The effect is specifically shown below:

Case 1

A male patient, 38 years old, with a 2×2 cm lump found on the right side of the neck, underwent lymphadenectomy and had a biopsy; immunohistochemistry: CK broad spectrum(-), EMA(-), LCA(+), CD20(+), CD79α(+), CD3(+), CD43(+), CD15(+), CD5(-), CD30(+), CD38(+), CD138(-), CD21(-), c-myc(+), ALK(-), Bcl2-2(weak+), Bcl6(-), Mum-1(+), PD-L1(+), Pax-5(-), Ki-67(+,60%), in situ hybridization: EBER(+), right cervical lymph node: ALK negative anaplastic large cell lymphoma suggested. Neck, chest and abdomen CT: multiple swollen lymph nodes were found in the supraclavicular fossa on both sides of the neck and the axilla. After clinical diagnosis, the patient was given the CHOEP regimen (specifically, 1 g of cyclophosphamide, 50 mg of adriamycin liposome, 2 mg of vincristine, 0.1 g of etoposide and 0.1 g of prednisone), 4 cycles of chemotherapy. Because the tumor was not completely relieved, the CHOEP-chidamide combination regimen was applied instead for chemotherapy, and chemotherapy was successfully performed. About 1 month after the patient finished the chemotherapy with the CHOEP-chidamide combination regimen, the preauricular and posterior auricular lymph nodes on the left side were found swollen without significant cause-the disease progressed. After enrollment, tablets of the compound of formula I were administered: 15 mg of the compound of formula I each day, 21 days as one cycle. The baseline SPD (sum of products of maximum vertical diameters of multiple lesions) was 717 mm. The SPD was 495 mm after 2 cycles of medication and 409 mm after 4 cycles of medication. Baseline PET-CT: the SUVmax (maximum standard uptake value) of the mediastinum was 1.9, the SUVmax of the liver was 2.6, and the SUVmax of the highest uptake lesion, the left parotid gland area, was 7.0. After 4 cycles of medication, PET-CT results showed that the SUVmax of the mediastinum was 1.5, the SUVmax of the liver was 2.5, and the SUVmax of the highest uptake lesion, the left parotid gland area, was 1.5. The overall therapeutic effect was complete response (CR), and no new lesion was found.

Case 2

A female patient, 33 years old; pathological diagnosis suggested: (left nasal cavity) extranodal NK/T cell lymphoma, nasal type; immunohistochemistry suggested tumor cells: CK(-), CD20(+), CD2(+), CD3(+), CD4(-), CD8(-), CD43(+), CD56(+), GranzymeB(+), TIA-1(+), Ki-67(30%+), EBER/ISH(+).

After clinical diagnosis, the patient was treated with the COEP-L regimen (4 mg of vindesine, 1.2 g of cyclophosphamide, 3750 IU of pegaspargase, 0.1 g of etoposide, and 15 mg of dexamethasone). After about four weeks, the SMILE regimen (adjusted according to the clinical practical medication to 40 mg of dexamethasone, 3 g of methotrexate, 2.3 g of ifosfamide, 3750 IU of pegaspargase, and 0.15 g of etoposide) was applied instead. After about one month, the P-GEMOX regimen (3750 IU of pegaspargase injection + 1.9 g of gemcitabine hydrochloride for injection + 60 mL of oxaliplatin injection) was applied instead. After about five weeks, AOEP chemotherapy + the G-CSF regimen were applied instead. After about six weeks, the SMILE regimen (40 mg of dexamethasone, 3 g of methotrexate, 2.3 g of ifosfamide, 3750 IU of pegaspargase, and 0.15 g of etoposide) was applied instead, and meanwhile, drugs (10 mg of methotrexate, 5 mg of dexamethasone, and 50 mg of cytarabine) were administered intrathecally. After about three weeks, autologous hematopoietic stem cell transplantation was performed. About 2 months after the autologous hematopoietic stem cell transplantation, tablets of chidamide were administered for about 4 consecutive months: 2 days per week, 1 dose per day, and 4 tablets per dose. CT results before enrollment suggested the disease progression.

After enrollment, tablets of the compound of formula I were administered: 15 mg of the compound of formula I each day. Before treatment, enhanced MRI of the paranasal sinuses showed: the SPD of measurable target lesions (the anterior part and lateral wall of the right nasal cavity) was 280 mm, the liver was normal, and the vertical diameter of the spleen was 12 cm. After administration, enhanced MRI examinations were performed regularly. After 2 cycles of treatment, the SPD decreased to 117 mm by 58.2%; the therapeutic effect was evaluated as partial response (PR), the liver was normal, the vertical diameter of the spleen was 11.5 cm, and no new lesion was found.

## Claims

1. A compound of formula **I** or a pharmaceutically acceptable salt thereof for use in treating peripheral T-cell lymphoma:

I

2. The compound of formula **I** or the pharmaceutically acceptable salt thereof according to claim 1, wherein the peripheral T-cell lymphoma is selected from relapsed or refractory peripheral T-cell lymphoma; optionally, the disease reoccurs after the patient with the peripheral T-cell lymphoma has been treated with a prior treatment regimen and achieved objective response, or the patient with the peripheral T-cell lymphoma has been treated with a prior treatment regimen but achieved no objective response.

3. The compound of formula **I** or the pharmaceutically acceptable salt thereof according to claim 1 or 2, wherein the peripheral T-cell lymphoma is selected from the group consisting of peripheral T-cell lymphoma, not otherwise specified (PTCL-NOS); angioimmunoblastic T-cell lymphoma (AITL); anaplastic large cell lymphoma (ALCL); and extranodal NK/T cell lymphoma (NKTCL), nasal type;
optionally, the peripheral T-cell lymphoma, not otherwise specified is selected from the group consisting of a GATA3-overexpressing type, a TBX21-overexpressing type, and a cytotoxin-overexpressing genotype.

4. The compound of formula **I** or the pharmaceutically acceptable salt thereof according to any one of claims 1-3, wherein the patient with the peripheral T-cell lymphoma has been treated with one or more prior treatment regimens; optionally, the patient with the peripheral T-cell lymphoma has been treated with one, two, three, four or five prior treatment regimens.

5. The compound of formula **I** or the pharmaceutically acceptable salt thereof according to any one of claims 1-4, wherein, the patient with the peripheral T-cell lymphoma is a patient who has been treated with one or more prior

treatment regimens comprising pegaspargase or L-asparaginase.

**6.** The compound of formula **I** or the pharmaceutically acceptable salt thereof according to claim 4, wherein the prior treatment regimens include drug therapies, radiotherapy or hematopoietic stem cell transplantation.

**7.** The compound of formula **I** or the pharmaceutically acceptable salt thereof according to claim 6, wherein the drug therapies include interferons, chemotherapy or targeted drug therapies; the radiotherapy is selected from the group consisting of total lymphoid irradiation and sub-total lymphoid irradiation; optionally, the radiotherapy includes involved field radiation therapy, involved nodal radiation therapy or involved site radiation therapy; the hematopoietic stem cell transplantation includes autologous hematopoietic stem cell transplantation or allogeneic hematopoietic stem cell transplantation.

**8.** The compound of formula **I** or the pharmaceutically acceptable salt thereof according to claim 7, wherein drugs used for the drug therapies are selected from one of pegaspargase, asparaginase (e.g., L-asparaginase), cyclophosphamide, ifosfamide, vincristine, vindesine, prednisone, prednisolone, doxorubicin, adriamycin, epirubicin, dexamethasone, methotrexate, cytarabine, carboplatin, cisplatin, bendamustine, fludarabine, mitoxantrone, etoposide, procarbazine, gemcitabine, methylprednisolone, methylprednisolone sodium succinate, mesna, oxaliplatin, 5-fluorouracil, azacitidine, pralatrexate, romidepsin, belinostat, chidamide, bortezomib, lenalidomide, thalidomide, calcium folinate, rituximab, genolimzumab, cemiplimab, pembrolizumab, nivolumab, sintilimab, tislelizumab, avelumab, atezolizumab and G-CSF, or combinations of more than one of the drugs described above.

**9.** The compound of formula **I** or the pharmaceutically acceptable salt thereof according to any one of claims 1-8, wherein an administration cycle for treating the peripheral T-cell lymphoma is 2-6 weeks.

**10.** The compound of formula **I** or the pharmaceutically acceptable salt thereof according to any one of claims 1-9, wherein a daily dose for treating the peripheral T-cell lymphoma is selected from 1-100 mg.

**11.** The compound of formula **I** or the pharmaceutically acceptable salt thereof according to any one of claims 1-10, wherein the number of daily administrations for treating the peripheral T-cell lymphoma is 1, 2 or 3.

**12.** Use of a compound of formula **I** or a pharmaceutically acceptable salt thereof for preparing a medicament for treating peripheral T-cell lymphoma

I

.

**13.** Use of a compound of formula **I** or a pharmaceutically acceptable salt thereof in treating peripheral T-cell lymphoma

I

.

**14.** A pharmaceutical composition for use in treating peripheral T-cell lymphoma, wherein the pharmaceutical composition comprises a compound of formula **I** or a pharmaceutically acceptable salt thereof

I

15. A method of treating peripheral T-cell lymphoma, wherein the method comprises administering to a patient an effective amount of a compound of formula **I** or a pharmaceutically acceptable salt thereof, or a pharmaceutical composition thereof

I

**INTERNATIONAL SEARCH REPORT**

International application No. **PCT/CN2021/118417**

**A. CLASSIFICATION OF SUBJECT MATTER**

A61K 31/519(2006.01)i； A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNTXT, CNABS, CNKI, DWPI, WOTXT, USTXT, EPTXT, PUBMED, STNext: 正大天晴, 封帆, 王训强, 赵颖, 韩溪, 陈莉, 马瑞婷, 吴乃营, 吡啶并[1,2-a]嘧啶酮, 磷脂酰肌醇3-激酶, 磷脂酰肌醇3激酶, 磷酸酯酰肌醇3激酶, 外周T细胞淋巴瘤, 淋巴瘤, pyrido[1,2-a]pyrimidone, phosphatidylinositol, PI3K, peripheral T-cell lymphoma, PTCL, 1845777-61-8

**C. DOCUMENTS CONSIDERED TO BE RELEVANT**

| Category* | Citation of document, with indication, where appropriate, of the relevant passages | Relevant to claim No. |
|---|---|---|
| X | WO 2017101847 A1 (CHIA TAI TIANQING PHARMACEUTICAL GROUP CO., LTD.) 22 June 2017 (2017-06-22)<br>abstract, and description, p. 1, compound 1, p. 3, second and third to last line, p. 4, compound 2, and pp. 16 and 17, test example 3 | 1-11, 14 |
| Y | WO 2017101847 A1 (CHIA TAI TIANQING PHARMACEUTICAL GROUP CO., LTD.) 23 December 2015 (2015-12-23)<br>abstract, and description, p. 1, compound 1, p. 3, second and third to last line, p. 4, compound 2, and pp. 16 and 17, test example 3 | 12, 13, 15 |
| X | WO 2015192760 A1 (MEDSHINE DISCOVERY INC.) 23 December 2015 (2015-12-23)<br>abstract, and description, p. 28, compound 25 in the table, and tables 3 and 4 | 1-11, 14 |
| Y | WO 2015192760 A1 (MEDSHINE DISCOVERY INC.) 23 December 2015 (2015-12-23)<br>abstract, and description, p. 28, compound 25 in the table, and tables 3 and 4 | 12, 13, 15 |
| Y | HUANG, Dachuan et al. "Evaluation of the PIK3 pathway in peripheral T-cell lymphoma and NK/T-cell lymphoma"<br>*BRITISH JOURNAL OF HAEMATOLOGY,* Vol. 189, 31 January 2020 (2020-01-31), pp. 731-744 | 12, 13, 15 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **26 November 2021** | **22 December 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/118417**

**C. DOCUMENTS CONSIDERED TO BE RELEVANT**

| Category* | Citation of document, with indication, where appropriate, of the relevant passages | Relevant to claim No. |
|---|---|---|
| Y | HUEN, A. et al. "Phase I/Ib Study of Tenalisib (RP6530), a Dual PI3Kδ/γ Inhibitor in Patients with Relapsed/Refractory T-Cell Lymphoma" *Cancers,* Vol. 12, 15 August 2020 (2020-08-15), pp. 1-14 | 12, 13, 15 |
| A | 刘晓健等 (LIU, Xiaojian et al.). "外周T 细胞性淋巴瘤药物治疗进展 (Treatment of Peripheral T-cell Lymphoma:New Drug Development)" *中国肿瘤影像学 (Oncoradiology),* Vol. 2, No. 3, 31 December 2009 (2009-12-31), pp. 83-86 | 1-15 |
| A | 代群 等, (DAI, Qun et al.). "Syk、PIK3、Akt在外周T细胞淋巴瘤中的表达及其意义 (Expression and Relationship of Syk, Pi3k and Akt in Peripheral T-Cell Lymphoma)" *安徽医科大学学报 (Acta Universitatis Medicinalis Anhui),* Vol. 49, No. 8, 31 August 2014 (2014-08-31), pp. 1141-1145 | 1-15 |

Form PCT/ISA/210 (second sheet) (January 2015)

**INTERNATIONAL SEARCH REPORT**

International application No.
**PCT/CN2021/118417**

**Box No. II Observations where certain claims were found unsearchable (Continuation of item 2 of first sheet)**

This international search report has not been established in respect of certain claims under Article 17(2)(a) for the following reasons:

1. [✓] Claims Nos.: **13,15**
because they relate to subject matter not required to be searched by this Authority, namely:

[1] Claim 13 sets forth a use of a compound of formula I or a pharmaceutically acceptable salt thereof in the treatment of peripheral T-cell lymphoma. Claim 15 sets forth a method for treating peripheral T-cell lymphoma. The subject matter of claims 13 and 15 relates to a method for treating diseases, and does not comply with PCT Rule 39.1(iv). The search report is made on the basis that the subject matter of claim 13 is amended to a "use of the compound of formula I a pharmaceutically acceptable salt thereof in the preparation of drugs for treating peripheral T-cell lymphoma", and the subject matter of claim 15 is amended to a "use of an effective amount of the compound of formula I or a pharmaceutically acceptable salt thereof, or a pharmaceutical composition thereof in the preparation of drugs for treating peripheral T cell lymphoma".

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2021/118417**

| Patent document cited in search report | Publication date (day/month/year) | Patent family member(s) | Publication date (day/month/year) |
|---|---|---|---|
| WO 2017101847 A1 | 22 June 2017 | EP 3395817 A1 | 31 October 2018 |
| | | ZA 201804007 B | 29 May 2019 |
| | | AU 2016369835 B2 | 15 October 2020 |
| | | RU 2018125475 A | 16 January 2020 |
| | | RU 2018125475 A3 | 28 February 2020 |
| | | CA 3008689 A1 | 22 June 2017 |
| | | AU 2016369835 A1 | 19 July 2018 |
| | | US 10479789 B2 | 19 November 2019 |
| | | US 2018319799 A1 | 08 November 2018 |
| | | CN 108602815 A | 28 September 2018 |
| | | JP 2018537497 A | 20 December 2018 |
| | | CN 108602815 B | 28 July 2020 |
| | | EP 3395817 A4 | 07 August 2019 |
| | | KR 20180095565 A | 27 August 2018 |
| | | HK 1256044 A1 | 13 September 2019 |
| WO 2015192760 A1 | 23 December 2015 | EA 032642 B1 | 28 June 2019 |
| | | CN 106470992 A | 01 March 2017 |
| | | KR 20170016465 A | 13 February 2017 |
| | | AU 2015276699 A1 | 19 January 2017 |
| | | EP 3159341 A1 | 26 April 2017 |
| | | EP 3159341 B8 | 08 January 2020 |
| | | TW I628180 B | 01 July 2018 |
| | | JP 2017519821 A | 20 July 2017 |
| | | EA 201790016 A1 | 30 June 2017 |
| | | CA 2951971 A1 | 23 December 2015 |
| | | EP 3159341 A4 | 16 May 2018 |
| | | ES 2754264 T3 | 16 April 2020 |
| | | TW 201625612 A | 16 July 2016 |
| | | US 2017137420 A1 | 18 May 2017 |
| | | EP 3159341 B1 | 21 August 2019 |
| | | JP 6680774 B2 | 15 April 2020 |
| | | AU 2015276699 B2 | 10 October 2019 |
| | | US 9856256 B2 | 02 January 2018 |
| | | CN 106470992 B | 06 November 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010967168
- WO 2015192760 A